**Europäisches Patentamt**

**European Patent Office**

**Office européen des brevets**

(19)

(11) Veröffentlichungsnummer: **0 084 019**
**A1**

(12) **EUROPÄISCHE PATENTANMELDUNG**

(21) Anmeldenummer: **83810003.0**

(22) Anmeldetag: **05.01.83**

(51) Int. Cl.³: **A 61 N 1/42**
**H 03 K 3/45**

(30) Priorität: **08.01.82 CH 81/82**

(43) Veröffentlichungstag der Anmeldung:
**20.07.83 Patentblatt 83/29**

(84) Benannte Vertragsstaaten:
**AT BE CH DE FR GB IT LI LU NL SE**

(71) Anmelder: **LKH AG**
**Frongartenstrasse 8**
**CH-9001 St. Gallen(CH)**

(72) Erfinder: **Resele, Rolf**
**Oberstrasse 100**
**CH-9000 St. Gallen(CH)**

(72) Erfinder: **Kellenberger, Rudolf**
**Seestrasse 29**
**CH-9403 Goldach(CH)**

(72) Erfinder: **Kurer, Walter**
**Rietbergstrasse 60**
**CH-9403 Goldach(CH)**

(74) Vertreter: **White, William et al,**
**c/o Patentanwaltsbureau ISLER & SCHMID**
**Walchestrasse 23**
**CH-8006 Zürich(CH)**

(54) **Gerät zur Erzeugung von magnetischen Impulsen.**

(57) Das Gerät umfasst als wesentlichen Baustein einen astabilen Multivibrator (M2) mit zwei einzeln in den Rückkopplungspfad einschaltbaren Kondensatoren (C2,C3) zur wahlweisen Erzeugung von Frequenzen im α-Wellenbereich und im θ-Wellenbereich des EEG. Ueber einen Leistungsverstärker (T) wird eine Spule (L) im Takt dieser Schwingungen geladen und entladen. Damit die magnetischen Impulse aus der Spule (L) steile Anstiegsflanken haben, ist ein zusätzlicher Kondensator (C5) zur Vergrösserung der Kapazität der Stromquelle (B) vorhanden. Die Dauer der Impulse wird durch einen Kondensator (C4) in der Basiszuleitung des Leistungsverstärkers (T) begrenzt. Die derart erzeugten magnetischen Impulse haben dieselbe Wirkung auf den menschlichen Körper wie die am zweiten Bioklimatologischen Kolloquium angegebenen Wirkungen von magnetischen Impulsen.

EP 0 084 019 A1

0084019

Gerät zur Erzeugung von magnetischen Impulsen

Die vorliegende Erfindung betrifft ein Gerät zur Erzeugung von magnetischen Impulsen gemäss dem Oberbegriff des unabhängigen Patentanspruchs 1.

Am zweiten Bioklimatologischen Kolloquium im September 1976 in München wurde über Untersuchungen berichtet, die unter der Leitung von Prof. Dr.med. H.U. Riethmüller an der Universität Thübingen durchgeführt wurden. Eine Anzahl Patienten mit psychosomatischen Beschwerden wurden dem Einfluss magnetischer Impulse ausgesetzt und deren Reaktion beobachtet. Es zeigte sich, dass bei Frequenzen im Bereich 1 - 15 Hz rheumatische Schmerzen und im Bereich 4 - 12 Hz insbesondere psychosomatische Beschwerden und Schmerzzustände reduziert werden konnten.

Aus dem Gebiet des "Biofeedback" gemäss dem Buch gleichen Namens von Wilfrid J. Hume,im Verlag Hans Huber, Bern, 1979 erschienen, ist es aus mehreren Untersuchungen bekannt geworden, dass Probanden ihre $\alpha$-Wellen des EEG verstärken oder abschwächen und dadurch die Schmerzfühligkeit verändern können. Insbesondere haben R. Melzach und C. Perry in einem Aufsatz "self regulation of pain" in der Zeitschrift Experimental Neurology, New York, 1975, Seiten 452-469, über die Möglichkeit berichtet, dass bei Probanden mit Kopfschmerzen die Schmerzen durch rückkoppelnde Beeinflussung der $\alpha$-Wellen verringert werden könnten.

Es ist ferner bekannt, dass Gehirnströme Wellen mit unterschiedlichen signifikanten Frequenzen im EEG erzeugen, nämlich $\alpha$-Wellen im Frequenzbereich 8 - 13 Hz, $\beta$-Wellen im Frequenzbereich 15 - 30 Hz, $\theta$-Wellen im Frequenzbereich 4 - 7,5 Hz und A-Wellen im Frequenzbereich 1 - 3 Hz. Obwohl aus der Medizin keine endgültigen Resultate aus der Gehirnforschung über die gegenseitigen Beziehungen zwischen Empfindungen und diesen Gehirnströmen vorliegen, hat nun die Untersuchung von Prof. Dr. Riethmüller gezeigt, dass von aussen einwirkende Wellen auf Schmerzen einen Einfluss haben, und zwar sollen die Wellen im Frequenzbereich der $\alpha$-Wellen liegen, also eine Frequenz zwischen 8 - 13 Hz haben.

Es ist demgemäss eine Aufgabe der Erfindung, ein Gerät zu schaffen, mit dem magnetische Impulse erzeugt werden können, um mit diesen Beschwerden und Schmerzen zu lindern.

Erfindungsgemäss wird dies durch die Merkmale im kennzeichnenden Teil des unabhängigen Patentanspruchs 1 erreicht.

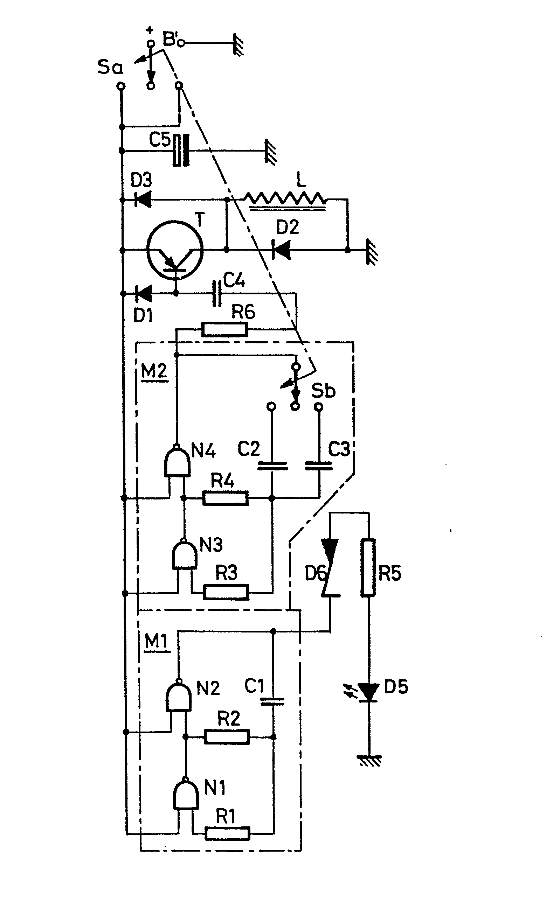

Nachfolgend wird die Erfindung anhand eines Ausführungsbeispiels erläutert. In der Zeichnung ist ein Schaltungsschema dargestellt.

Im Schaltungsschema lassen sich ein erster astabiler Multivibrator M1, ein zweiter astabiler Multivibrator M2, eine Verstärkerstufe T, eine Spule L und ein Speiseteil B, Sa, C5 erkennen.

Die je einen Impulsgenerator bildenden astabilen Multivibratoren M1, M2 sind je mit zwei UND-Toren mit invertiertem Ausgang N1, N2 und N3, N4 aufgebaut. Von den UND-Toren ist je ein Eingang mit der positiven Speisespannungsleitung verbunden und am zweiten Eingang ist ein Widerstand R1, R2 und R3, R4 angeschlossen. Die Ausgänge der steuernden UND-Tore N1, N3 sind auf die Eingänge der nachfolgenden kippenden UND-Tore N2, N4 geführt und die Ausgänge der kippenden UND-Tore N2, N4 sind über Kondensatoren C1, C2, C3 an die freien
Anschlüsse der genannten Widerstände R1, R2, R3, R4 zurückgeführt.

Somit bilden je ein Kondensator C1; C2 oder C3 und ein Widerstand R2, R4 die zeitbestimmenden Glieder der astabilen Multivibratoren M1, M2.

Die Ausgänge der kippenden UND-Tore N2, N4 sind gleichzeitig
auch die Ausgänge der Multivibratoren. Der Ausgang des ersten
Multivibrators M1 ist über eine Zener-Diode D6, einen Widerstand R5 und eine Leucht-Diode D5 auf Masse geschaltet. Mit
einem Widerstandswert von 47 M$\Omega$ und einer Kapazität von 100
nF für den Widerstand R2 und den Kondensator C1 schwingt der
erste Multivibrator mit einer Frequenz von angenähert 1 Hz.
Mit der Zenerdiode D6 wird erreicht, dass die Leucht-Diode
D5 bei ungenügender Speisespannung nicht aufleuchtet und so
anzeigt, dass das Gerät nicht betriebsbereit ist. Der Widerstand R5 bildet eine Strombegrenzung für die Leucht-Diode D5.

Der Ausgang des zweiten Multivibrators M2 ist über den Basiswiderstand R6 und einen Ladekondensator C4 auf die Basis des
Transistors T geführt, in dessen Kollektorzuleitung die
Spule L geschaltet ist. Die Dioden D1, D2 und D3 sind Schutzdioden für den Transistor T, um Spannungsspitzen beim Ein-
und Ausschalten der Spule L zu verhindern. Mit einer Kapazität von 10 nF für den Kondensator C3 und einem Widerstandswert von 4,7 M$\Omega$ für den Widerstand R4 ergibt sich eine
Frequenz von 4,7 Hz und von 22 nF für den Kondensator C2
eine solche von 10,3 Hz. Mit dem Kondensator C4 mit einer
Kapazität von 15 nF wird die Impulsdauer auf etwa 1 ms Dauer
begrenzt, nämlich auf die Zeit, die es braucht, diesen Kondensator C4 zu laden und damit den Transistor T zu sperren.

Die Speisung erfolgt aus einer Batterie B, deren positiver
Pol + die positive Speisspannung bildet und deren negativer
Pol - auf Masse geschaltet ist. Der Kondensator C5 dient zur
Erhöhung der Leistung der Batterie B zur Abgabe eines genügend hohen Stromes beim Einschalten der Spule 4.

Wie dargestellt, besteht der Schalter S aus zwei Schalterebenen Sa und Sb, mit denen ausser dem Einschalten des Gerätes auch die Frequenzen umgeschaltet werden können, derart,
dass nur ein einziger Schalter zu betätigen ist.

Ausgedehnte Versuche haben gezeigt, dass mit diesem Gerät
vergleichbare Ergebnisse erzielt werden konnten, wie sie anlässlich des zweiten Bioklimatologischen Kolloquiums bekannt
gegeben wurden.

P a t e n t a n s p r ü c h e

1. Gerät zur Erzeugung magnetischer Impulse im Frequenzbereich 1 bis 30 Hz, gekennzeichnet durch eine Schaltungsanordnung mit wenigstens einem Impulsgenerator (M1,M2) und eine über einen Leistungsverstärker (T) von diesem angesteuerte Spule (L).

2. Gerät nach Patentanspruch 1, dadurch gekennzeichnet, dass der Impulsgenerator (M1,M2) aus einem ersten UND-Tor mit invertiertem Ausgang (N1;N3) und aus einem zweiten UND-Tor mit invertiertem Ausgang (N2;N4) gebildet ist, bei denen das zeitbestimmende Glied (C1,R2;C2,C3,R4) im Rückkopplungspfad zwischen dem Ausgang des zweiten UND-Tores (N2;N4) und dessen einem Eingang geschaltet ist.

3. Gerät nach Patentanspruch 2, dadurch gekennzeichnet, dass ein erster Impulsgenerator (M1) zur Abgabe von Kontroll-Impulsen und ein zweiter Impulsgenerator (M2) zur Ansteuerung der Spule (L) vorhanden sind.

4.      Gerät nach Patentanspruch 3, dadurch gekennzeichnet, dass das zeitbestimmende Glied des zweiten Impulsgebers
(M2) wenigstens zwei einzeln in den Rückkopplungspfad einschaltbare Kondensatoren (C2,C3) aufweist.

5.      Gerät nach Patentanspruch 1, dadurch gekennzeichnet, dass der Leistungsverstärker ein Transistor (T) mit
der Spule (L) in der Kollektorzuleitung ist, und dass in die
Basiszuleitung in Reihe zum Basiswiderstand (R6) ein Kondensator (C4) geschaltet ist.

6.      Gerät nach Patentanspruch 4, dadurch gekennzeichnet, dass zur Erzeugung von Impulsen mit einer Impulswiderholungsfrequenz im $\alpha$-Wellenbereich des EEG das zeitbestimmende Glied einen Widerstand (R4) von 4,7 M$\Omega$ und einen Kondensator (C3) von 10 nF aufweist.

7.      Gerät nach Patentanspruch 4, dadurch gekennzeichnet, dass zur Erzeugung von Impulsen mit einer Impulswiderholungsfrequenz im $\theta$-Wellenbereich des EEG das zeitbestimmende Glied einen Widerstand (R4) von 4,7 M$\Omega$ und einen Kondensator (C2) von 22 nF aufweist.

8.      Gerät nach Patentanspruch 6 oder 7, dadurch gekennzeichnet, dass die Spule (L) zur Abgabe von magnetischen Impulsen mit einer Feldstärke in der Grössenordnung von 200 A/m eingerichtet ist.

Europäisches Patentamt

# EUROPÄISCHER RECHERCHENBERICHT

| EINSCHLÄGIGE DOKUMENTE | | EP 83810003.0 |
|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | CH – A5 – 570 172 (ESB)<br>* Spalte 5, Zeilen 31-55; Fig. 1,2 *<br>-- | 1,5 | A 61 N 1/42<br>H 03 K 3/45 |
| A | EP – A1 – 0 042 889 (ELECTRO-BIOLOGY)<br>* Seite 10, Zeilen 6-21; Fig. 5 *<br>-- | 1-3 | |
| A | DE – A1 – 2 553 197 (BUSCHKY)<br>* Seite 4, Zeile 11 – Seite 5, Zeile 12; Fig. *<br>-- | 1 | |
| X | US – A – 3 497 712 (JUNGCLAS)<br>* Spalte 4, Zeilen 4-22; Fig. 1 *<br>---- | 1-3 | |

| RECHERCHIERTE SACHGEBIETE (Int. Cl. 3) |
|---|
| A 61 N<br>H 03 K |

Der vorliegende Recherchenbericht wurde für alle Patentansprüche erstellt.

| Recherchenort | Abschlußdatum der Recherche | Prüfer |
|---|---|---|
| WIEN | 12-04-1983 | NEGWER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82